# EUROPEAN PATENT APPLICATION

(11) **EP 4 179 996 A1**
(43) Date of publication of application: **17.05.2023**
(21) Application number: 21837507.9
(22) Date of filing: 22.06.2021
(51) Int. Cl.: A61B 34/30

(54) **ROBOTIC SYSTEM AND EXIT METHOD**

(30) Priority: 10.07.2020 CN 202010663761
(71) Applicant: Beijing Surgerii Technology Co., Ltd., Haidian Beijing 100192 (CN)
(72) Inventor: XU, Kai, Beijing 100192 (CN); GAO, Guorong, Beijing 100192 (CN)
(74) Representative: karo IP
(86) International application number: PCT/CN2021/101654
(87) International publication number: WO 2022/007628

(57) **Abstract**

A robot system (100) includes a manipulator assembly (30), a driver assembly (20), and a controller (10), where the manipulator assembly (30) is disposed on the driver assembly (20), the driver assembly (20) is configured to drive the manipulator assembly (30) to move, the controller (10) is communicatively connected to the driver assembly (20), and the controller (10) is configured to control, based on a fault type of the robot system (100), the driver assembly (20) to drive the manipulator assembly (30) to exit from a current pose. A user may adopt different exit modes according to fault types and adopt different fault handling processes according to fault emergency levels, so as to implement an accurate and efficient fault exist process.

## Description

### Cross Reference to Related Applications

This application claims priority to Chinese Patent Application No. 2020106637610 filed on July 10, 2020 and entitled "Surgical robot and exit method for surgical robot", which is incorporated herein by reference in its entirety.

### Technical Field

The present disclosure relates to the field of robots, and in particular, to a robot system and an exit method.

### Background Art

For a medical device product, the principle of "safety after failure" is generally used to deal with system faults. It is required that a system is set to a safety state in time after the system detects a fault, and an alarm signal is sent to prompt a user, so that the user may clear the fault according to the alarm prompt.

For a surgical robot system, when it is determined that a fault occurs, a tool arm needs to exit from a human body for ending an operation, so as to help the medical staff to carry out follow-up patient treatment. However, currently, when there is no differentiation between fault types, according to a safety requirement, motion of all components of the surgical robot needs to be stopped, and the tool arm needs to be manually handled to exit. This process is complicated, and is therefore time-consuming and laborious, which is not conducive to rapid ending of the operation, thereby affecting quick treatment of patients.

### Summary of the Invention

In some embodiments, the present disclosure provides a robot system, including: a manipulator assembly; a driver assembly on which the manipulator assembly is disposed, where the driver assembly is configured to drive the manipulator assembly to move; and a controller communicatively connected to the driver assembly and configured to control, based on a fault type of the robot system, the driver assembly to drive the manipulator assembly to exit from a current pose.

In some embodiments, the present disclosure provides an exit method for a robot system, including: determining a fault type based on fault information of the robot system; determining whether the fault type is a first-type fault; and controlling, in response to the fact that the fault type is the first-type fault, a driver assembly to drive a manipulator assembly to exit from a current pose.

### Brief Description of the Drawings

To describe technical solutions in embodiments of the present disclosure clearly, the following briefly describes the accompanying drawings required for describing the embodiments of the present disclosure. The accompanying drawings in the following descriptions show merely some embodiments of the present disclosure, and those of ordinary skill in the art may still derive other embodiments from the content of the embodiments of the present disclosure and these accompanying drawings without creative efforts.
FIG. 1 is a structural block diagram of a robot system according to some embodiments of the present disclosure; and
FIG. 2 is a flowchart of an exit method for a robot system according to some embodiments of the present disclosure.

### Detailed Description of the Embodiments

To make the resolved technical problems, used technical solutions, and achieved technical effects of the present disclosure more clearly, the technical solutions in embodiments of the present disclosure are further described in detail below with reference to the accompanying drawings. Apparently, the described embodiments are merely exemplary rather than all of the embodiments of the present disclosure.

In descriptions of the present disclosure, it should be noted that, direction or position relationships indicated by terms such as "central", "upper", "lower", "left", "right", "vertical", "horizontal", "inner", and "outer" and the like are direction or position relationships based on the accompanying drawings, and are merely intended to facilitate the descriptions of the present disclosure and simplify the descriptions, rather than indicating or implying that a referred apparatus or element must have a particular direction or be constructed and operated in a particular direction. Therefore, these terms should not be interpreted as limiting the present disclosure. In addition, the terms "first" and "second" are for descriptive purposes only, and should not be construed as indicating or implying relative importance. It should be noted that, in the descriptions of the present disclosure, unless expressly specified and limited otherwise, the terms "mounted", "connected to each other", "connected to", and "coupled" should be understood in a broad sense. For example, "connection" may be a fixed connection, a detachable connection, a mechanical connection, or an electrical connection; or may be a direct connection or an indirect connection by means of an intermediate medium; or may be an internal communication between two elements. For those of ordinary skill in the art, specific meanings of the foregoing terms in the present disclosure may be understood based on specific situations. In the present disclosure, in a surgical robot system, an end closer to a user (for example, a surgeon) is defined as a nearer end or a nearer part or a rear end or a rear part, and an end closer to a surgical patient is defined as a farther end or a farther part or a front end or a front part. Those skilled in the art may understand that the embodiments of the present disclosure may be applied to medical devices or surgical robots, and may also be applied to other non-medical apparatuses.

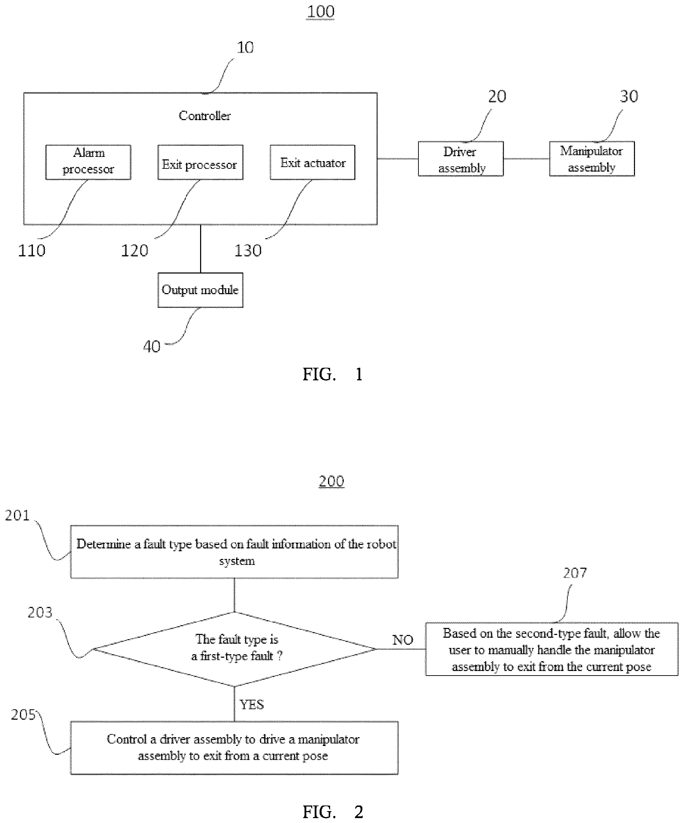

The present disclosure provides a robot system. FIG. 1 is a structural block diagram of a robot system 100 according to some embodiments of the present disclosure. As shown in FIG. 1, the robot system 100 may include a manipulator assembly 30, a driver assembly 20, and a controller 10. The manipulator assembly 30 is of a mechanical structure that may be operated by the controller 10. In some embodiments, the manipulator assembly 30 may include one or more surgical tools and/or endoscopes. The surgical tool and/or the endoscope may include a surgical tool arm and an end device. It should be understood that the surgical tool arm may include a flexible surgical tool arm, for example, a flexible continuum structure. The end device of the surgical tool may include, but is not limited to, forceps, curved scissors, and the like. The end device of the endoscope may include, but is not limited to, an illumination apparatus or an image acquisition apparatus.

In some embodiments, the manipulator assembly 30 is disposed on the driver assembly 20, for example, may be disposed at a farther end of the driver assembly 20. The driver assembly 20 is configured to drive the manipulator assembly 30 to move. Those skilled in the art should understand that, the driver assembly 20 may include a plurality of motors and a sensor coupled to the motor. The sensor may include, but is not limited to, an encoder or a potentiometer. In some embodiments, the manipulator assembly 30 is coupled to the motor of the driver assembly 20. The controller 10 may control the motor of the driver assembly 20 to perform motion, to drive the manipulator assembly 30 to move. The sensor of the driver assembly 20 may be connected to the controller 10 through a cable connection or wireless connection. The sensor may acquire parameters of the motor of the driver assembly 20 and connection status information of the driver assembly 20 and the manipulator assembly 30, and send the parameters of the motor and the connection status information to the controller 10. The controller 10 may monitor an operating status of the driver assembly 20 in real time based on the parameters of the motor, and acquire a current pose of an end device of the manipulator assembly 30. The current pose of the end device may include a current position and a current orientation. For example, a pose of the end device may include an axial feed position of the end device along the manipulator assembly 30 and a rotation and deflection angle at the position.

In some embodiments, communication between the controller 10 and the driver assembly 20 may include communication based on a drive command (for example, a command for controlling motion parameters of the motor) and communication based on status information (for example, status information of the motor and status information of the manipulator assembly 30). The communication based on a drive command and the communication based on status information may be performed over different transmission channels. In this way, if a drive command transmission channel between the controller 10 and the driver assembly 20 is abnormal, the controller 10 cannot transmit the drive command to the driver assembly 20, and the driver assembly 20 may send the status information of the motor and the status information of the manipulator assembly 30 to the controller 10 over a status information transmission channel, so that the controller 10 can acquire the status information of the motor and the manipulator assembly 30 in real time and monitor the motor and the manipulator assembly 30.

In some embodiments, the controller 10 is communicatively connected to the driver assembly 20. The controller 10 is configured to control, based on a fault type of the robot system 100, the driver assembly 20 to drive the manipulator assembly 30 to exit from the current pose. In some embodiments, if a fault occurs in the robot system 100 in an actual operation process of the robot system 100 (for example, during a surgery), the controller 10 may control, based on a fault type of the robot system 100, the motor of the driver assembly 20 to perform motion, so as to control the manipulator assembly 30 to exit from the current pose. The fault of the robot system 100 may include a fault in hardware or software constituting the robot system 100. It should be understood that the current pose may include an effective position and orientation in which the manipulator assembly 30 can perform surgical operations, or may include a real-time pose of the manipulator assembly 30.

In some embodiments, the fault type of the robot system 100 may include a first-type fault and a second-type fault. In some embodiments, the first-type fault may include a recoverable non-communication fault and an unrecoverable non-communication fault that are unrelated to a normal communication function of the driver assembly 20. In some embodiments, for example, the recoverable non-communication fault of the first-type fault may include, but is not limited to, faults such as breaking of a contact of an adapter connected to a sterile protective sleeve and an abnormality in a connection between the manipulator assembly and the driver assembly. In some embodiments, the unrecoverable non-communication fault of the first-type fault may include, but is not limited to, a fault in a pedal of a main console cart of the robot system 100, a fault in a main manipulator of the robot system 100, and the like. The first-type fault is unrelated to a normal communication function of the driver assembly 20. It should be understood that when the first-type fault occurs in the robot system 100, the controller 10 may still control the driver assembly 20 to perform motion, to control the manipulator assembly 30 to exit.

In some embodiments, the controller 10 may be configured to control, based on the recoverable non-communication fault, the driver assembly 20 to drive the manipulator assembly 30 to exit from the current pose. For example, the controller 10 may automatically control, based on the recoverable non-communication fault, the driver assembly 20 to drive the manipulator assembly 30 to exit from the current pose, or receive an input command from a user, and control, based on the input command, the driver assembly 20 to drive the manipulator assembly 30 to exit from the current pose. In some embodiments, the controller 10 is configured to receive, based on the unrecoverable non-communication fault, an input command from the user, and control, based on the input command, the driver assembly 20 to drive the manipulator assembly 30 to exit from the current pose. It should be understood that the input command may be input by the user via a user interface. The user interface may include, but is not limited to, a keyboard, a touch screen, a button, a microphone, and the like.

In some embodiments, the input command may include an orientation command and an exit command. The orientation command is used to cause the manipulator assembly 30 to be in an exit orientation, and the exit command is used to cause the manipulator assembly 30 to exit. It should be understood that the manipulator assembly 30 may be in a non-straight orientation during the actual operation. The driver assembly 20 may control, based on the orientation command, the manipulator assembly 30 to be in the exit orientation. The exit orientation may include a straight orientation, a partially straight orientation, a partially arc orientation, or an orientation matched with a shape of a catheter sheath. After the manipulator assembly 30 moves to be in the exit orientation, the driver assembly 20 may control, based on the exit command, the manipulator assembly 30 to exit from the exit orientation. In some embodiments, the input command may alternatively include only the exit command. The driver assembly 20 may control, based on the exit command, the manipulator assembly 30 to directly exit from the exit orientation. It should be understood that the orientation command and the exit command may be input by means of a single input command, or may be input by means of a plurality of input commands.

In some embodiments, the second-type fault may include a communication fault that affects the normal communication function of the driver assembly 20. For example, the second-type fault may include, but is not limited to, a communication interruption, a fault in the motor of the driver assembly 20, a power fault in the motor of the driver assembly 20, a fault in the sensor of the driver assembly 20, or a fault in software related to motion control of the driver assembly 20. It should be understood that the communication fault in the present disclosure should be interpreted in a broad sense. The second-type fault may directly affect the normal communication function of the driver assembly 20, or indirectly affect normal communication of the driver assembly 20, for example, the driver assembly 20 cannot perform motion normally. For example, due to the fault in the sensor of the driver assembly 20 or the fault in the software related to motion control of the driver assembly 20, the controller 10 cannot effectively control the driver assembly 20, which indicates that a communication fault occurs. Alternatively, due to the fault in the motor or the power fault in the motor of the driver assembly 20, the driver assembly 20 cannot execute a drive command of the controller 10, which indicates that a communication fault occurs. It should be understood that when the second-type fault occurs in the robot system 100, due to the communication fault of the driver assembly 20, the controller 10 cannot control the driver assembly 20 to perform motion, such that the manipulator assembly 30 cannot exit from the current pose. In some embodiments, the controller 10 may be configured to send, based on the communication fault, communication fault alarm information, and allow a user to manually handle the manipulator assembly 30 to exit from the current pose.

In some embodiments, the controller 10 may be configured to send, based on the first-type fault, first-type alarm information, and send, based on the second-type fault, second-type alarm information. In some embodiments, the first-type alarm information may include recoverable non-communication fault alarm information and unrecoverable non-communication fault alarm information. The controller 10 is configured to send, based on the recoverable non-communication fault, the recoverable non-communication fault alarm information, and send, based on the unrecoverable non-communication fault, the unrecoverable non-communication fault alarm information.

In some embodiments, the robot system 100 may further include an output module 40. It should be understood that the output module 40 may be communicatively connected to the controller 10, and may be configured to output the first-type alarm information and/or the second-type alarm information. In some embodiments, the output module 40 may include, but is not limited to, at least one of an audio output module, a light output module, or an image output module. For example, the audio output module may include a speaker or a voice broadcast device. The light output module may include a light strip, and light strips with different colors may display different alarm information. For example, based on that the fault type is the first fault, the first-type alarm information may be indicated in a blinking green light. Based on that the fault type is the second fault, the second-type alarm information may be indicated in a blinking yellow light. The image output module may include a display screen or a touch screen. The output module 40 enumerated in this embodiment of the present disclosure is exemplary rather than restrictive. It should be understood that the output module 40 may alternatively be another output form that can remind and warn the user, which all fall within the protection scope of the present disclosure. Those skilled in the art should understand that, alarm information of different types may be output by using the same or different output modules 40.

In some embodiments, the controller 10 includes: an alarm processor 110, an exit processor 120, and an exit actuator 130. The exit processor 120 may be communicatively connected to the alarm processor 110 and the exit actuator 130. In some embodiments, the alarm processor 110 may be configured to receive fault information of the robot system 100, determine the fault type based on the fault information, and send the first-type alarm information based on the first-type fault (for example, the recoverable non-communication fault and the unrecoverable non-communication fault), or send the second-type alarm information based on the second-type fault (for example, the communication fault).

In some embodiments, the exit processor 120 may be configured to determine an exit type based on the fault type. In some embodiments, the exit type may include automatic fault exit and manual fault exit. For example, the exit type may be determined as the automatic fault exit based on the recoverable non-communication fault and the unrecoverable non-communication fault. The exit type may be determined as the manual fault exit based on the communication fault. In some embodiments, the fault type may be obtained by querying for a fault information list. The robot system 100 may generate, based on the fault type, different fault information. The different fault information may correspond to the same or different exit types.

In some embodiments, the exit actuator 130 may determine a fault exit mode based on the exit type, and send a drive command to the driver assembly 20 based on the determined fault exit mode, or send an operation indication to an output module 40 based on the determined fault exit mode. It should be understood that, the exit actuator 130 may determine the fault exit mode based on the exit type, and control the motion of the driver assembly 20 by adopting different modes, to drive the manipulator assembly 30 to exit.

In some embodiments, the fault exit mode may include a first fault exit mode, a second fault exit mode, and a third fault exit mode. The first fault exit mode may be an automatic control mode, for example, may be used for the controller 10 to autonomously control the motion of the driver assembly 20 by means of a command. In some embodiments, the exit actuator 130 may be configured to determine the first fault exit mode based on the recoverable non-communication fault and the automatic fault exit, for example, a fault that does not affect the control over the driver assembly 20 by the controller 10, for example, a display error of the output module 40, and control, based on the first fault exit mode, the driver assembly 20 to drive the manipulator assembly 30 to exit from the current pose. It should be understood that when a fault occurs in the robot system 100, the controller 10 may determine a type of the fault, and in response to the fact that the fault is the recoverable non-communication fault, send a drive command to the driver assembly 20 by utilizing the first fault exit mode, to automatically control the driver assembly 20 to drive the manipulator assembly 30 to exit from the current pose. It should be understood that, based on the recoverable non-communication fault, it may be determined that the motor of the driver assembly 20 operates normally, and that the communication between the controller 10 and the driver assembly 20 is normal, and the drive command is sent in the first fault exit mode. For example, the drive command may include motor motion parameter information of the controller, so as to implement a rotation direction and angle of the manipulator assembly 30 through motor motion of the controller.

It should be understood that the second fault exit mode may be semi-automatic. For example, the controller 10 requires the user to input a command or manually control the driver assembly 20 to perform motion. For example, based on the unrecoverable non-communication fault and the automatic fault exit, the second fault exit mode is determined to be adopted. In some embodiments, the exit actuator 130 may be configured to send a first operation indication to the output module 40 based on the second fault exit mode and the unrecoverable non-communication fault and the automatic fault exit, to prompt the user to input a command, so as to control the driver assembly 20 to drive the manipulator assembly 30 to exit from the current pose. For example, when a fault occurs in the robot system 100, the controller 10 may determine a type of the fault, and in response to the fact that the fault is the unrecoverable non-communication fault, control, in the second fault exit mode based on the command input by the user, the driver assembly 20 to drive the manipulator assembly 30 to exit from the current pose. It should be understood that, based on the unrecoverable non-communication fault, it may be determined that the motor of the driver assembly 20 operates normally, and the first operation indication is sent to the output module 40 in the second fault exit mode. For example, the output module 40 may include an image display apparatus, for example, a touch screen. The first operation indication may be displayed on the touch screen.

In some embodiments, the first operation indication may prompt the user to complete the exit of the manipulator assembly 30, for example, display prompt information or display an exit control key on the touch screen. The user may trigger the exit control key according to the first operation indication, to control the driver assembly 20 to drive the manipulator assembly 30 to exit. It should be understood that the exit control key may further include an exit button. For example, the exit button may be disposed on the driver assembly 20 or another component of the robot system 100. The first operation indication may include prompting the user to press the exit button. The user may press the exit button to control the driver assembly 20 to drive the manipulator assembly 30 to exit. It should be understood that the exit control key may alternatively be disposed on a remote control device associated with the motor. In some embodiments, the exit control key may include an orientation button and an exit button. It should be understood that the orientation button may be triggered via a touch screen. The exit button may be triggered by pressing a button disposed on the driver assembly 20. In some embodiments, both the orientation button and the exit button may be triggered via the touch screen or by pressing the button. In some embodiments, the orientation button and the exit button may be integrated into a same button. The above embodiments are merely exemplary rather than restrictive. For example, when it is determined that a farther end of the manipulator assembly 30 is in a bending state, the user may press the orientation button, and the driver assembly 20 may receive an input command from the user, and control, based on the input command, the motor of the driver assembly 20 to perform motion, to handle the farther end of the manipulator assembly 30 to be in the exit orientation, for example, in a straight orientation. Then, the manipulator assembly 30 is handled to exit from the current pose. It should be understood that when it is determined that the farther end of the manipulator assembly 30 is already in the straight orientation, the user may alternatively input a command to the driver assembly 20 by directly triggering the exit button on the driver assembly 20, and control, based on the input command, the motor of the driver assembly 20 to perform motion, so as to directly handle the manipulator assembly 30 to exit from the current pose. Therefore, the manipulator assembly 30, for example, a surgical tool or an endoscope, is controlled to exit from a catheter sheath in a surgical operative area.

In some embodiments, when no fault occurs in the robot system 100, the user may alternatively control, based on an input command, for example, by pressing the exit control key, the motor of the driver assembly 20 to perform motion, so as to handle the manipulator assembly 30 to exit from the current pose.

In some embodiments, the third fault exit mode may be a manual control mode. For example, the controller 10 cannot automatically control, via a command, the driver assembly 20 to perform motion, and cannot control, based on the command input by the user, the driver assembly 20 to perform motion. The exit actuator 130 may alternatively be configured to send a second operation indication to the output module 40 in the third fault exit mode based on that the exit type is the manual fault exit, to prompt the user to manually handle the manipulator assembly 30 to exit from the current pose. For example, when a fault occurs in the robot system 100, the controller 10 may determine a type of the fault, and in response to the fact that the fault is the communication fault, send the second operation indication to the output module 40 in the third fault exit mode. The user may manually control, based on the second operation indication, the driver assembly 20 to drive the manipulator assembly 30 to exit from the current pose. It should be understood that, based on the communication fault, it may be determined that the communication between the controller 10 and the driver assembly 20 is abnormal or blocked, and the motor of the driver assembly 20 is in an abnormal state and cannot operate normally. In this case, the second operation indication is sent to the output module 40 in the third fault exit mode. For example, the output module 40 may include an image display apparatus, and the second operation indication may include an operation interface position of the manual manner and an indication of an operation manner. Based on the second operation indication, the user may manually, for example, physically disassemble the driver assembly 20, to handle the manipulator assembly 30 to exit from the current pose. For example, the user may manually rotate a motor bearing of the driver assembly 20 to drive the manipulator assembly 30 to move for exiting.

In some embodiments, the second operation indication may include a plurality of messages prompting the user to perform required operations. When it is determined that a distance between the current pose of the manipulator assembly 30 and a human tissue is less than a safety range, the second operation indication may prompt the user to drive, by using the driver assembly 20, the manipulator assembly 30 to move in a direction away from the human tissue. When the manipulator assembly 30 moves to a position at a distance, exceeding a preset distance, from the current pose and is within a safety range in which the manipulator assembly causes no injury to the human body, the second operation indication may prompt the user to manually disassemble the manipulator assembly 30 from the driver assembly 20. Based on that the farther end of the manipulator assembly 30 is in the bending state, the second operation indication may further prompt the user to manually disassemble the manipulator assembly 30 from the driver assembly 20, so as to handle the disassembled manipulator assembly 30 to be in the exit orientation, for example, in the straight orientation, so that the manipulator assembly is conveniently taken out from the surgical operative area, thereby rapidly ending the surgery.

In some embodiments of the present disclosure, the exit type is determined by using the controller 10, and a drive command is sent to the driver assembly 20 according to the exit type, to automatically handle the manipulator assembly 30 to exit, or an operation indication is sent to the output module 40 to cause the user to complete the exit of the manipulator assembly 30 according to the operation indication. Therefore, a better control effect and man-machine experience are achieved. Different exit modes are adopted according to different fault types, and different fault handling processes may be adopted according to fault emergency levels. An accurate and efficient fault exit process is implemented by integrating all non-manual operation steps into computer control. For steps that cannot be autonomously completed by using a computer, detailed operation prompts and operation indications may be provided for the user to handle faults with the simplest operation in a shorter time, so that the manipulator assembly 30 can conveniently and rapidly exit.

The present disclosure provides a control method for a robot system. FIG. 2 is a flowchart of a control method 200 for a robot system (for example, the robot system 100) according to some embodiments of the present disclosure. As shown in FIG. 2, the method 200 may be performed by a controller (for example, the controller 10) of the robot system 100. The controller 10 may be configured on a computing device. The method 200 may be implemented by software and/or hardware.

In step 201, a fault type is determined based on fault information of the robot system. It should be understood that the robot system 100 may include a plurality of components, for example, may include a main console cart and a patient-side cart. The main console cart may include the controller 10, an output module 40, and a main manipulator (not shown). The patient-side cart may include a driver assembly 20 and a manipulator assembly 30. When a hardware or software error or fault occurs in some of the components, fault information may be sent to the controller 10 according to a preset form, or the fault information may be generated when the controller 10 does not receive communication information from a corresponding component within a preset time period and/or fails to extract information. Different fault information may be classified into different types of fault information. Different types of fault information may respectively correspond to different fault types. In this way, a fault type of the robot system may be determined based on different fault information.

In step 203, whether the fault type is a first-type fault is determined. For example, different types of fault information may include first fault information and second fault information. The first fault information corresponds to the first-type fault, and the second fault information corresponds to a second-type fault. A severity level of the fault may be determined by determining whether the fault type is the first-type fault, so that the robot system and/or a user may take a fault handling measure based on the fault.

In step 205, a driver assembly is controlled to drive the manipulator assembly to exit from a current pose. For example, the driver assembly is automatically controlled to drive the manipulator assembly to exit from the current pose, or an input command from the user is received, and the driver assembly is controlled, based on the input command, to drive the manipulator assembly to exit from the current pose. In some embodiments, the first-type alarm information is sent based on that the fault type is the first-type fault. The first-type fault may include a recoverable non-communication fault and an unrecoverable non-communication fault that are unrelated to a normal communication function of the driver assembly 20. The controller 10 may automatically send a drive command to the driver assembly 20 in response to the fact that the fault type is the recoverable non-communication fault in the first-type fault, so as to control the driver assembly 20 to drive the manipulator assembly 30 to exit from the current pose. The controller 10 may receive the input command from the user in response to the fact that the fault type is the unrecoverable non-communication fault in the first-type fault, and control, based on the input command, the driver assembly 20 to drive the manipulator assembly 30 to exit from the current pose.

In some embodiments, the method 200 may further include step 207. In step 207, the user is allowed, based on the second-type fault, to manually handle the manipulator assembly to exit from the current pose. In some embodiments, the second-type alarm information is sent based on that the fault type is the second-type fault. The second-type fault may include a communication fault that affects a normal communication function of the driver assembly 20. The second-type alarm information is sent in response to the fact that the fault type is the second-type fault, and the user is allowed to manually control the driver assembly 20 to perform motion, so as to handle the manipulator assembly 30 to exit from the current pose.

In some embodiments, the method 200 may further include: determining an exit type based on the fault type, determining a fault exit mode based on the exit type, and controlling, based on the fault exit mode, the driver assembly to drive the manipulator assembly to exit. For example, the exit type may be determined as automatic fault exit based on the recoverable non-communication fault and the unrecoverable non-communication fault. The exit type may be determined as manual fault exit based on the communication fault. In some embodiments, based on that the exit type is the automatic fault exit including the recoverable non-communication fault, the driver assembly 20 is controlled, based on a first fault exit mode, to drive the manipulator assembly 30 to exit from the current pose. Based on that the exit type is the automatic fault exit including the unrecoverable non-communication fault, a first operation indication is sent to the output module 40 based on a second fault exit mode, such that the user inputs a command based on the first operation indication, to control the driver assembly 20 to drive the manipulator assembly 30 to exit from the current pose. Based on that the exit type is the manual fault exit, a second operation indication is sent to the output module 40 based on a third fault exit mode, and the user is allowed to manually handle the manipulator assembly 30 to exit from the current pose.

It should be noted that the foregoing descriptions are merely exemplary embodiments of the present disclosure and technical principles applied thereto. Those skilled in the art can understand that the present disclosure is not limited to the specific embodiments herein, and various obvious changes, readjustments, and substitutions can be made by those skilled in the art without departing from the protection scope of the present disclosure. Therefore, the present disclosure is described in detail by the foregoing embodiments, but the present disclosure is not limited to the foregoing embodiments. Other equivalent embodiments may also be included without departing from the concept of the present disclosure. Therefore, the scope of the present disclosure depends on the scope of the appended claims.

## Claims

1. A robot system, comprising:
a manipulator assembly;
a driver assembly on which the manipulator assembly is disposed, wherein the driver assembly is operable to drive the manipulator assembly to move; and
a controller communicatively connected to the driver assembly and configured to control, based on a fault type of the robot system, the driver assembly to drive the manipulator assembly to exit from a current pose.

2. The robot system according to claim 1, wherein the fault type comprises:
a first-type fault comprising a recoverable non-communication fault and an unrecoverable non-communication fault that are unrelated to a normal communication function of the driver assembly; and
a second-type fault comprising a communication fault that affects the normal communication function of the driver assembly.

3. The robot system according to claim 2, wherein
the controller is configured to send first-type alarm information based on the first-type fault; and
the controller is configured to send second-type alarm information based on the second-type fault.

4. The robot system according to claim 3, wherein
the first-type alarm information comprises recoverable non-communication fault alarm information and unrecoverable non-communication fault alarm information; and
the controller is configured to send the recoverable non-communication fault alarm information based on the recoverable non-communication fault, and send the unrecoverable non-communication fault alarm information based on the unrecoverable non-communication fault.

5. The robot system according to claim 3, further comprising:
an output module communicatively connected to the controller and configured to output the first-type alarm information and/or the second-type alarm information.

6. The robot system according to claim 2, wherein the controller is configured to control, based on the recoverable non-communication fault, the driver assembly to drive the manipulator assembly to exit from the current pose.

7. The robot system according to claim 2, wherein the controller is configured to receive an input command from a user based on the unrecoverable non-communication fault, and control, based on the input command, the driver assembly to drive the manipulator assembly to exit from the current pose.

8. The robot system according to claim 2, wherein the controller is configured to send communication fault alarm information based on the communication fault, and allow a user to manually handle the manipulator assembly to exit from the current pose.

9. The robot system according to claim 7, wherein the input command comprises an orientation command and an exit command, the orientation command is used for causing the manipulator assembly to be in an exit orientation, and the exit command is used for causing the manipulator assembly to exit.

10. The robot system according to claim 5, wherein the output module comprises at least one of an audio output module, a light output module, or an image output module.

11. The robot system according to claim 2, wherein the controller comprises:
an alarm processor configured to: receive fault information of the robot system, determine the fault type based on the fault information, and send first-type alarm information based on the recoverable non-communication fault and the unrecoverable non-communication fault or send second-type alarm information based on the communication fault;
an exit processor configured to determine an exit type based on the fault type; and
an exit actuator configured to determine a fault exit mode based on the exit type, and send a drive command to the driver assembly based on the determined fault exit mode or send an operation indication to an output module based on the determined fault exit mode.

12. The robot system according to claim 11, wherein the exit type comprises automatic fault exit and manual fault exit;
the exit type is determined as the automatic fault exit based on the recoverable non-communication fault and the unrecoverable non-communication fault; and
the exit type is determined as the manual fault exit based on the communication fault.

13. The robot system according to claim 12, wherein
the exit actuator is configured to determine a first fault exit mode based on the recoverable non-communication fault and the automatic fault exit, and control, based on the first fault exit mode, the driver assembly to drive the manipulator assembly to exit from the current pose; or
the exit actuator is configured to: determine a second fault exit mode based on the unrecoverable non-communication fault and the automatic fault exit; send a first operation indication for prompting a user to input a command to the output module based on the second fault exit mode, and control, based on the input command, the driver assembly to drive the manipulator assembly to exit from the current pose.

14. The robot system according to claim 13, wherein
the exit actuator is configured to determine a third fault exit mode based on the manual fault exit, and send a second operation indication to the output module based on the third fault exit mode, to prompt the user to manually handle the manipulator assembly to exit from the current pose.

15. The robot system according to claim 14, wherein the second operation indication comprises a plurality of messages for prompting the user to perform required operations.

16. An exit method for a robot system, comprising:
determining a fault type based on fault information of the robot system;
determining whether the fault type is a first-type fault; and
controlling, in response to the fact that the fault type is the first-type fault, a driver assembly to drive a manipulator assembly to exit from a current pose.

17. The exit method according to claim 16, wherein the first-type fault comprises a recoverable non-communication fault and an unrecoverable non-communication fault that are unrelated to a normal communication function of the driver assembly; and
the exit method comprises: controlling, in response to the fact that the fault type is the recoverable non-communication fault, the driver assembly to drive the manipulator assembly to exit from the current pose.

18. The exit method according to claim 16 or 17, further comprising:
sending first-type alarm information in response to the fact that the fault type is the first-type fault; and
sending second-type alarm information in response to the fact that the fault type is a second-type fault, wherein the second-type fault comprises a communication fault that affects the normal communication function of the driver assembly.

19. The exit method according to claim 17, further comprising:
receiving an input command from a user in response to the fact that the fault type is the unrecoverable non-communication fault; and
controlling, based on the input command, the driver assembly to drive the manipulator assembly to exit from the current pose.

20. The exit method according to claim 18, further comprising:
sending communication fault alarm information in response to the fact that the fault type is the communication fault, wherein the alarm information is used to prompt the user to manually handle the manipulator assembly to exit from the current pose.
